# DEMANDE DE BREVET EUROPEEN

(11) **EP 1 420 030 A2**
(43) Date de publication de la demande: **19.05.2004**
(21) Numéro de dépôt: 03293085.1
(22) Date de dépôt: 10.12.2003
(51) Int. Cl.: C07K 5/06

(54) **Procédé de synthèse de dérivés de l'acide (2S, 3aS, 7aS) - 1 - [(S)-alanyl]-octahydro-1H-indole-2-carboxylique et application à la synthèse du perindorpil**

(71) Demandeur: Les Laboratoires Servier, 92415 Courbevoie Cedex (FR)
(72) Inventeur: Dubuffet, Thierry, 76190 Autretot (FR); Lecouve, Jean-Pierre, 76600 Le Havre (FR)

(57) **Abrégé**

Procédé de synthèse des composés de formule (I) : 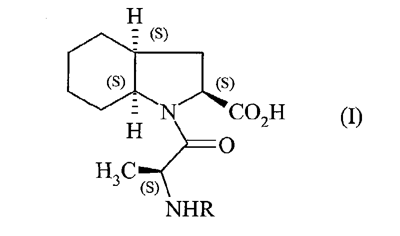 dans laquelle R représente un atome d'hydrogène ou un groupement protecteur de la fonction amino.

Application à la synthèse du perindopril et de ses sels pharmaceutiquement acceptables.

## Description

La présente invention concerne un procédé de synthèse des composés de formule (I) : 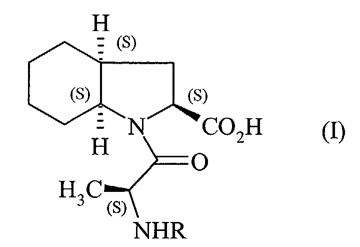 dans laquelle R représente un atome d'hydrogène ou un groupement protecteur de la fonction amino,
et leur application à la synthèse du perindopril de formule (II) : 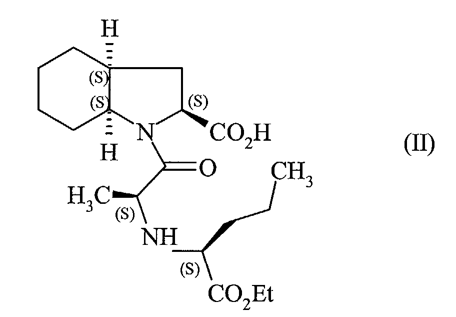 et de ses sels pharmaceutiquement acceptables.

Le perindopril, ainsi que ses sels pharmaceutiquement acceptables, et plus particulièrement son sel de tert-butylamine, possèdent des propriétés pharmacologiques intéressantes.
Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir y accéder avec un procédé de synthèse performant, facilement transposable à l'échelle industrielle, conduisant au perindopril avec un bon rendement et une excellente pureté.
Le brevet EP 0 308 341 décrit la synthèse industrielle du perindopril par couplage de l'ester benzylique de l'acide (2*S*, 3a*S*, 7a*S*)-octahydroindole 2-carboxylique avec l'ester éthylique de la N-[(*S*)-1-carboxybutyl]-(*S*)-alanine en présence de dicyclohexylcarbodiimide, suivie de la déprotection du groupement carboxylique de l'hétérocycle par hydrogénation catalytique.
Ce procédé présente des inconvénients liés à l'utilisation du dicyclohexylcarbodiimide.

La Demanderesse a mis au point un procédé de synthèse du perindopril qui utilise d'autres agents de couplage.

Plus spécifiquement, la présente invention concerne un procédé de synthèse du perindopril
caractérisé en ce que l'on met en réaction l'ester benzylique de formule (IIIa) ou (IIIb) : 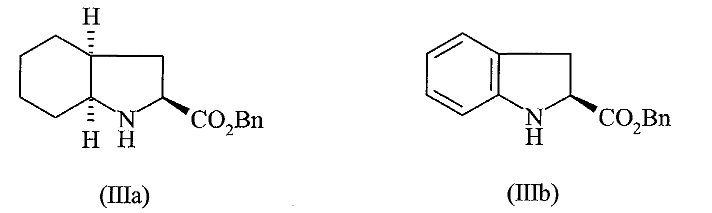 ou le sel d'addition de l'ester de formule (IIIa) ou (IIIb) avec un acide minéral ou organique,
avec le dérivé de l'alanine de formule (IV) : 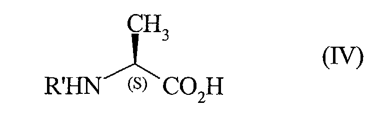 dans laquelle R' représente un groupement protecteur de la fonction amino,
en présence d'un agent de couplage choisi parmi les réactifs et couples de réactifs suivants :
(1,3-diméthylaminopropyl)-3-éthyl-carbodiimide chlorhydrate,
(1,3-diméthylaminopropyl)-3-éthyl-carbodiimide chlorhydrate / 1-hydroxybenzotriazole,
(1,3-diméthylaminopropyl)-3-éthyl-carbodiimide chlorhydrate/1-hydroxy-7-azabenzotriazole,
(1,3-diméthylaminopropyl)-3-éthyl-carbodiimide chlorhydrate / N-hydroxysuccinimide,
(1,3-diméthylaminopropyl)-3-éthyl-carbodiimide chlorhydrate /3-hydroxy-3,4-dihydro-4-oxo-1,2,3-benzotriazine,
(1,3-diméthylaminopropyl)-3-éthyl-carbodiimide chlorhydrate / N-hydroxyphtalimide,
dicyclohexylcarbodiimide / 1-hydroxy-7-azabenzotriazole,
dicyclohexylcarbodiimide / N-hydroxysuccinimide,
dicyclohexylcarbodiimide /3-hydroxy-3,4-dihydro-4-oxo-1,2,3-benzotriazine,
dicyclohexylcarbodiimide / N-hydroxyphtalimide,
O-(benzotriazol-1-yl)-1,1,3,3-tétraméthyluronium hexafluorophosphate,
O-(7-azabenzotriazol-1-yl)-1,1,3,3-tétraméthyluronium hexafluorophosphate,
O-(benzotriazol-1-yl)-1,1,3,3-tétraméthyluronium tétrafluoroborate,
benzotriazol-1-yl-oxytripyrrolidinophosphonium hexafluorophosphate,
benzotriazol-1-yl-oxy-tris-(diméthylamino)-phosphonium hexafluorophosphate,
O-(benzotriazol-1-yl)-1,1,3,3-bis(tétraméthylène)-uronium hexafluorophosphate,
O-(benzotriazol-1-yl)-1,1,3,3-bis(pentaméthylène)-uronium hexafluorophosphate,
chloro-tripyrrolidinophosphonium hexafluorophosphate,
chloro-1,1,3,3-bis(tétraméthylène)-formamidinium hexafluorophosphate,
chloro-1,1,3,3-bis(pentaméthylène)-formamidinium hexafluorophosphate,
N-éthoxycarbonyl-2-éthoxy-1,2-dihydroquinoléine,
O-[(éthoxycarbonyl)-cyanométhylènamino]-1,1,3,3- tétraméthyluronium tétrafluoroborate,
O-(3,4-dihydro-4-oxo-1,2,3-benzotriazin-3-yl-1,1,3,3-tétraméthyluronium tétrafluoroborate,
O-(3,4-dihydro-4-oxo-1,2,3-benzotriazin-3-yl-1,1,3,3-tétraméthyluronium tétrafluoroborate /1-hydroxybenzotriazole,
O-(3,4-dihydro-4-oxo-1,2,3-benzotriazin-3-yl-1,1,3,3-tétraméthyluronium tétrafluoroborate /N-méthylmorpholine,
O-(3,4-dihydro-4-oxo-1,2,3-benzotriazin-3-yl-1,1,3,3-tétraméthyluronium tétrafluoroborate /collidine,
O-(1,2-dihydro-2-oxo-1-pyridyl)-1,1,3,3-tétraméthyluronium tétrafluoroborate,
O-(1,2-dihydro-2-oxo-1-pyridyl)-1,1,3,3-tétraméthyluronium tétrafluoroborate/1-hydroxybenzotriazole,
O-(1,2-dihydro-2-oxo-1-pyridyl)-1,1,3,3-bis(tétraméthylène)-uronium hexafluorophosphate,
O-(1,2-dihydro-2-oxo-1-pyridyl)-1,1,3,3-bis(tétraméthylène)-uronium hexafluorophosphate/1-hydroxy-benzotriazole,
O-(N-succinimidyl)-1,1,3,3-tétraméthyluronium tétrafluoroborate,
O-(N-succinimidyl)-1,1,3,3-bis(tétraméthylène)uronium tétrafluoroborate,
O-(N-succinimidyl)-1,1,3,3-bis(tétraméthylène)uronium tétrafluoroborate/1-hydroxybenzotriazole,
O-(5-norbornène-2,3-dicarboximido)-1,1,3,3-tétraméthyluronium tétrafluoroborate, anhydride propanephosphonique,
imide de l'acide N-hydroxy-5-norbornène-2,3-dicarboxylique,
et N-hydroxy-1,2-dihydro-2-oxo-pyridine,
en présence éventuelle de base,
pour conduire respectivement au composé de formule (Va) ou (Vb), selon que l'on est parti du composé de formule (IIIa) ou (IIIb) : 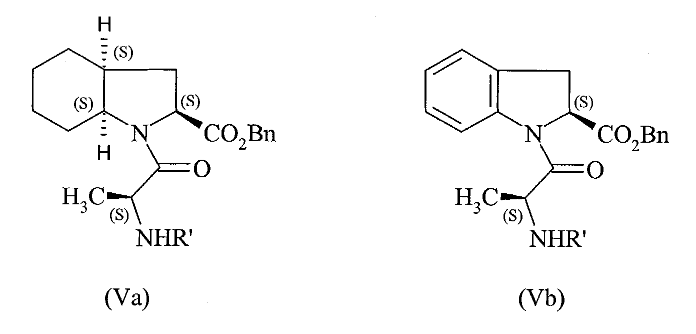 dans laquelle R' est tel que défini précédemment,
que l'on soumet à une réaction d'hydrogénation catalytique en présence de palladium, pour conduire au produit de formule (I).

Parmi les groupements protecteurs de la fonction amino utilisables dans la présente invention, on peut citer à titre non limitatif les groupements tert-butyloxycarbonyle, benzyle et benzyloxycarbonyle.

L'hydrogénation catalytique du composé de formule (Va) est préférentiellement effectuée sous une pression d'hydrogène inférieure à 10 bars.

L'hydrogénation catalytique du composé de formule (Vb) est préférentiellement effectuée sous une pression d'hydrogène comprise entre 10 et 35 bars.

Le composé de formule (I) ainsi obtenu est ensuite soumis, le cas échéant, à une réaction de déprotection de la fonction amino, suivie d'une réaction de couplage, soit avec le 2-oxopentanoate d'éthyle dans des conditions d'amination réductrice,
soit avec un composé de formule (VI) : 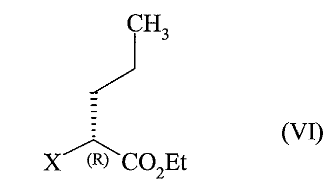 dans laquelle X représente un groupement partant choisi parmi atome d'halogène,
―O―SO₂CH₃ et 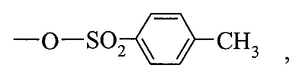 pour conduire au perindopril optiquement pur, que l'on transforme, si on le souhaite, en un sel pharmaceutiquement acceptable tel que le sel de tert-butylamine.

Les exemples ci-dessous illustrent l'invention.

### Exemple 1 : Acide (2S, 3aS, 7aS)-1-{(2S)-2-[(tert-butyloxycarbonyl)-amino]-propionyl}-octahydro-1H-indole-2-carboxylique / méthode 1 :

### Stade A : (2S, 3aS, 7aS)-1-{(2S)-2-[(Tert-butyloxycarbonyl)-amino]-propionyl}-octahydro-1H-indole-2-carboxylate de benzyle :

Dans un réacteur sous agitation sont introduits 200 g du paratoluènesulfonate de l'ester benzylique de l'acide (2*S*, 3a*S*, 7a*S*)-octahydroindole 2-carboxylique, 65 ml de triéthylamine, 1 1 d'acétate d'éthyle puis, après 10 mn d'agitation à température ambiante, 87 g de N-[tert-butyloxycarbonyl]-(*S*)-alanine, et 175 g de O-(benzotriazol-1-yl)-1,1,3,3-bis(tétraméthylène)-uronium hexafluorophosphate. Le mélange hétérogène est ensuite porté à 30°C pendant 3h sous bonne agitation, puis il est refroidi à 0°C et filtré.
Le filtrat est ensuite lavé, puis évaporé à sec pour conduire au produit attendu.

### Stade B : Acide (2S, 3aS, 7aS)-1-{(2S)-2-[(tert-butyloxycarbonyl)-amino]-propionyl}-octahydro-1H-indole-2-carboxylique :

Le résidu obtenu dans le stade précédent (200 g) est mis en solution dans 200 ml de méthylcyclohexane et transféré dans un hydrogénateur, puis 26 g de charbon palladié à 5% en suspension dans 80 ml de méthylcyclohexane sont ajoutés, suivis de 640 ml d'eau.
Le mélange est ensuite hydrogéné sous une pression de 0,5 bar, à une température comprise entre 15 et 30°C, jusqu'à absorption de la quantité théorique d'hydrogène.
Après filtration du catalyseur, la phase aqueuse du filtrat est lavée par du méthylcyclohexane, puis lyophilisée pour conduire au produit attendu avec un rendement de 94%.

### Exemple 2 : Acide (2S, 3aS, 7aS)-1-{(2S)-2-[(tert-butyloxycarbonyl)-amino]-propionyl}-octahydro-1H-indole-2-carboxylique / méthode 2 :

### Stade A : (2S)-1-{(2S)-2-[(Tert-butyloxycarbonyl)-amino]-propionyl}-2,3-dihydro-1H-indole-2-carboxylate de benzyle :

Dans un réacteur sous agitation sont introduits 200 g du paratoluènesulfonate du 2,3-dihydro-1*H*-indole-2-carboxylate de benzyle, 66 ml de triéthylamine, 1 1 d'acétate d'éthyle puis, après 10 mn d'agitation à température ambiante, 89 g de N-[tert-butyloxycarbonyl]-(*S*)-alanine, et 151 g de O-(benzotriazol-1-yl)-1,1,3,3-bis(tétraméthylène)-uronium tétrafluoroborate. Le mélange hétérogène est ensuite porté à 30°C pendant 3h sous bonne agitation, puis il est refroidi à 0°C et filtré.
Le filtrat est ensuite lavé, puis évaporé à sec pour conduire au produit attendu.

### Stade B : Acide (2S, 3aS, 7aS)-1-{(2S)-2-[(tert-butyloxycarbonyl)-amino]-propionyl}-octahydro-1H-indole-2-carboxylique :

Le résidu obtenu dans le stade précédent (200 g) est mis en solution dans 200 ml de méthylcyclohexane et transféré dans un hydrogénateur, puis 26 g de charbon palladié à 5% en suspension dans 80 ml de méthylcyclohexane sont ajoutés, suivis de 640 ml d'eau.
Le mélange est ensuite hydrogéné sous une pression de 0,5 bar, à une température comprise entre 15 et 30°C, jusqu'à absorption de la quantité théorique d'hydrogène nécessaire à la débenzylation, puis le mélange est porté à une température comprise entre 50 et 100°C et hydrogéné sous une pression de 30 bars, jusqu'à absorption de la quantité théorique d'hydrogène nécessaire à l'hydrogénation du cycle.
Après filtration du catalyseur, la phase aqueuse du filtrat est lavée par du méthylcyclohexane, puis lyophilisée pour conduire au produit attendu.

## Revendications

1. Procédé de synthèse des composés de formule (I) 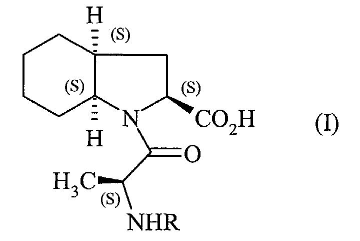 dans laquelle R représente un atome d'hydrogène ou un groupement protecteur de la fonction amino,
**caractérisé en ce que** l'on met en réaction l'ester benzylique de formule (IIIa) ou (IIIb) : 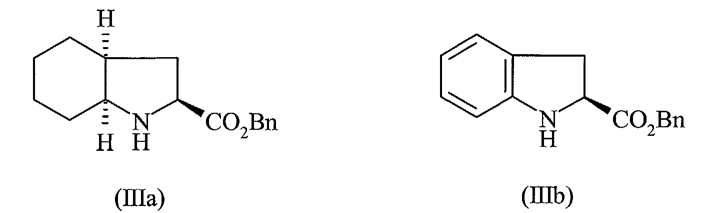 ou le sel d'addition de l'ester de formule (IIIa) ou (IIIb) avec un acide minéral ou organique,
avec le dérivé de l'alanine de formule (IV) : 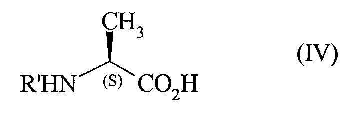 dans laquelle R' représente un groupement protecteur de la fonction amino,
en présence d'un agent de couplage choisi parmi les réactifs et couples de réactifs suivants :
(1,3-diméthylaminopropyl)-3-éthyl-carbodiimide chlorhydrate,
(1,3-diméthylaminopropyl)-3-éthyl-carbodiimide chlorhydrate / 1-hydroxybenzotriazole,
(1,3-diméthylaminopropyl)-3-éthyl-carbodiimide chlorhydrate/1-hydroxy-7-azabenzotriazole,
(1,3-diméthylaminopropyl)-3-éthyl-carbodiimide chlorhydrate / N-hydroxysuccinimide,
(1,3-diméthylaminopropyl)-3-éthyl-carbodiimide chlorhydrate /3-hydroxy-3,4-dihydro-4-oxo-1,2,3-benzotriazine,
(1,3-diméthylaminopropyl)-3-éthyl-carbodiimide chlorhydrate / N-hydroxyphtalimide,
dicyclohexylcarbodiimide / 1-hydroxy-7-azabenzotriazole,
dicyclohexylcarbodiimide / N-hydroxysuccinimide,
dicyclohexylcarbodiimide /3-hydroxy-3,4-dihydro-4-oxo-1,2,3-benzotriazine,
dicyclohexylcarbodiimide / N-hydroxyphtalimide,
O-(benzotriazol-1-yl)-1,1,3,3-tétraméthyluronium hexafluorophosphate,
O-(7-azabenzotriazol-1-yl)-1,1,3,3-tétraméthyluronium hexafluorophosphate,
O-(benzotriazol-1-yl)-1,1,3,3-tétraméthyluronium tétrafluoroborate,
benzotriazol-1-yl-oxytripyrrolidinophosphonium hexafluorophosphate,
benzotriazol-1-yl-oxy-tris-(diméthylamino)-phosphonium hexafluorophosphate,
O-(benzotriazol-1-yl)-1,1,3,3-bis(tétraméthylène)-uronium hexafluorophosphate,
O-(benzotriazol-1-yl)-1,1,3,3-bis(pentaméthylène)-uronium hexafluorophosphate,
chloro-tripyrrolidinophosphonium hexafluorophosphate,
chloro-1,1,3,3-bis(tétraméthylène)-formamidinium hexafluorophosphate,
chloro-1,1,3,3-bis(pentaméthylène)-formamidinium hexafluorophosphate,
N-éthoxycarbonyl-2-éthoxy-1,2-dihydroquinoléine,
O-[(éthoxycarbonyl)-cyanométhylènamino]-1,1,3,3- tétraméthyluronium tétrafluoroborate,
O-(3,4-dihydro-4-oxo-1,2,3-benzotriazin-3-yl-1,1,3,3-tétraméthyluronium tétrafluoroborate,
O-(3,4-dihydro-4-oxo-1,2,3-benzotriazin-3-yl-1,1,3,3-tétraméthyluronium tétrafluoroborate /1-hydroxybenzotriazole,
O-(3,4-dihydro-4-oxo-1,2,3-benzotriazin-3-yl-1,1,3,3-tétraméthyluronium tétrafluoroborate /N-méthylmorpholine,
O-(3,4-dihydro-4-oxo-1,2,3-benzotriazin-3-yl-1,1,3,3-tétraméthyluronium tétrafluoroborate /collidine,
O-(1,2-dihydro-2-oxo-1-pyridyl)-1,1,3,3-tétraméthyluronium tétrafluoroborate,
O-(1,2-dihydro-2-oxo-1-pyridyl)-1,1,3,3-tétraméthyluronium tétrafluoroborate/1-hydroxybenzotriazole,
O-(1,2-dihydro-2-oxo-1-pyridyl)-1,1,3,3-bis(tétraméthylène)-uronium hexafluorophosphate,
O-(1,2-dihydro-2-oxo-1-pyridyl)-1,1,3,3-bis(tétraméthylène)-uronium hexafluorophosphate/1-hydroxy-benzotriazole,
O-(N-succinimidyl)-1,1,3,3-tétraméthyluronium tétrafluoroborate,
O-(N-succinimidyl)-1,1,3,3-bis(tétraméthylène)uronium tétrafluoroborate,
O-(N-succinimidyl)-1,1,3,3-bis(tétraméthylène)uronium tétrafluoroborate/1-hydroxybenzotriazole,
O-(5-norbornène-2,3-dicarboximido)-1,1,3,3-tétraméthyluronium tétrafluoroborate, anhydride propanephosphonique,
imide de l'acide N-hydroxy-5-norbornène-2,3-dicarboxylique,
et N-hydroxy-1,2-dihydro-2-oxo-pyridine,
en présence éventuelle de base,
pour conduire respectivement au composé de formule (Va) ou (Vb), selon que l'on est parti du composé de formule (IIIa) ou (IIIb) : 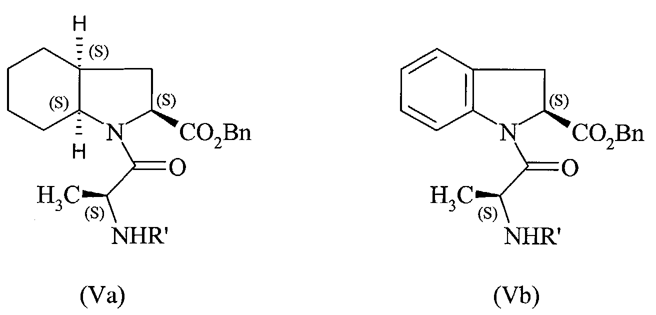 dans laquelle R' est tel que défini précédemment,
que l'on soumet à une réaction d'hydrogénation catalytique en présence de palladium, pour conduire au produit de formule (I).

2. Procédé selon la revendication 1, **caractérisé en ce que** l'on part du composé de formule (IIIa).

3. Procédé selon la revendication 1, **caractérisé en ce que** l'on part du composé de formule (IIIb).

4. Procédé selon la revendication 2, **caractérisé en ce que** la réaction d'hydrogénation du composé de formule (Va) est effectuée sous une pression d'hydrogène inférieure à 10 bars.

5. Procédé selon la revendication 3, **caractérisé en ce que** la réaction d'hydrogénation du composé de formule (Vb) est effectuée sous une pression d'hydrogène comprise entre 10 et 35 bars.

6. Procédé de synthèse du perindopril ou de ses sels pharmaceutiquement acceptables à partir du composé de formule (I), **caractérisé en ce que** le composé de formule (I) est obtenu par le procédé de synthèse selon l'une quelconque des revendications 1 à 5.
